# EUROPEAN PATENT SPECIFICATION

(11) **EP 1 185 200 B1**
(45) Date of publication and mention of the grant of the patent: **09.01.2008**
(21) Application number: 00938168.2
(22) Date of filing: 05.06.2000
(51) Int. Cl.: A61B 5/107, A61M 25/01

(54) **Indicia for an endoscopic medical device**
Markierungen für eine medizinische endoskopische Vorrichtung
Indices destinés à un dispositif médical endoscopique

(30) Priority: 05.06.1999 US 137824 P
(43) Date of publication of application: 13.03.2002
(73) Proprietor: WILSON-COOK MEDICAL INC., Winston-Salem, North Carolina 27105 (US)
(72) Inventor: HOLLAND-CLARK, Tamisha, A., Pfafftown, NC 27040 (US); HAWKINS, Melvin, Kem, Bloomington, IN 47401 (US); KARPIEL, John, A., Winston-Salem, NC 27106 (US)
(74) Representative: Jehan, Robert
(86) International application number: PCT/US2000/015532
(87) International publication number: WO 2000/074565

(56) References cited:
- EP-A- 0 519 214
- EP-A- 0 723 786
- WO-A-96/39077
- US-A- 5 209 730
- US-A- 5 320 602

## Description

### Technical Field

This invention relates to minimally medical devices, more particularly, wire guides and related devices.

### Background of the Invention

Endoscopy provides a less-invasive means of examining and treating a number of areas of the body that are accessible through a natural body opening. Scopes have been designed for placement through the mouth, rectum, or genitourinary tract, etc. for performing procedures at more remote locations, such as the lungs, gastrointestinal tract, and bladder. Certain procedures, such as an Endoscopic Retrograde Cholangiopancreatography (ERCP) have been specifically developed to take advantage of this technology. The purpose of an ERCP procedure is to gain access to the biliary system to either evaluate jaundice when duct blockage is suspected, to diagnose cancer, or to locate strictures or masses in pancreatic ducts, liver, and biliary tree. This is accomplished by advancing a catheter through the scope and into the biliary tree where dye is injected under fluoroscopic imaging.

When strictures are found within a duct, the physician usually wants to obtain a measurement, e.g., for purposes of placing a stent to maintain the lesion open. While the endoscope is designed for viewing structures within the body, the biliary tree does not provide a favorable environment for direct observation. Thus, a stricture or mass must be assessed by a more indirect means. Fluoroscopy measurement may be possible, but it is often difficult to obtain an accurate measurement using radiographic techniques. This can be critical such as when placing a stent, where the size selected must equal or exceed the length of the constriction. What is needed is a non-invasive method for measuring strictures and similar structures in the biliary tree and other areas of the body that is simple, fast, and accurate. A secondary goal is to be able to obtain these measurements by utilizing devices that are already part of the procedure.

European Patent Application EP0723786A1 discloses an catheter apparatus for measuring lesions in a blood vessel, the apparatus comprising first elongate member and a second member slidably disposed inside the first member. Each member include a radiopaque marker which is aligned with one of the boundaries of the lesion. The length of the lesion by measuring the distance between the makers, using calibration markings located on the second member, which may extend to the proximal portion of the second member which directly visible to the operator. A lumen within the inner second member allows the catheter apparatus to be directed to the target site over a wire guide. The document US-A-5333620 discloses a medical device according to the preamble of claim 1.

### Summary of the Invention

The foregoing problems are solved and a technical advance is achieved in an illustrative embodiment of an exchange wire guide, such as a solid nitinol core wire with a polymer outer coating, such as PTE, that is used with an endoscope, and has at least a first system of indicia located thereon, comprising scale indicia for measuring anatomical distances within a body of a patient. The scale indicia can include a series of sequentially increasing markings, such as bands, a numerical scale system, or both. Other types of non-numerical indicia can be included as well, such as other symbols or colors to indicate relative distances to a fixed point on the device, which may be the distal end or some other point (e.g., the 200 cm mark on a ERCP wire guide). In one embodiment, used with an endoscope for ERCP, the scale indicia are located within a region that is 200 to 230 cm from the distal end of the wire guide, a portion of the wire guide that at least partially protrude from the proximal end of an endoscope when the wire guide is placed within the biliary system.

In the invention, the medical device includes a second system of indicia for indicating relative motion of the medical device to endoscope. The second system of indicia, which may include diagonal lines, helical striping, etc, that is printed on or incorporated into the medical device, helps the operator determine if the medical device has longitudinally shifted in position, such as during an exchange procedure, thus preventing dislodgement. The second system of indicia can be located about the proximal portion of the wire guide where it is directly visible to the operator, or located about the distal portion of the wire guide where it can be viewed by the endoscope within the patient.

One primary advantage of the illustrative embodiment is the ability to measure anatomical structures when the wire guide is used with an endoscope having an accessory channel for introducing ancillary devices or instrumentation. It is especially useful for ERCP (Endoscopic Retrograde Cholangiopancreatography) procedures where, for example, a stricture is to be measured. In an ERCP procedure, the endoscope is advanced into the duodenum where the wire guide is then advanced from the distal end of the endoscope into the Papilla of Vater to access the biliary system. To measure the stricture, the wire guide having a radiopaque distal portion is advanced until it has crossed the stricture. The wire guide tip is made visible by the use of a radiopaque material, either by having the polymer outer coating comprise a radiopaque material; adding a polymer tip such as PEBAX^{®} (Elf Atochem North America, Philadelphia, PA), which is loaded with a radiopaque powder, such as tungsten; addition of a second material, such as radiopaque bands or shrink tubing; or making the wire itself radiopaque, such as by placement of a platinum coil over a tapered solid core wire. In one aspect of the invention, the clinician determines the position of the wire guide as it exits the proximal or exposed end of the endoscope or catheter, then withdraws the wire guide until the radiopaque tip marks the proximal boundary of the stricture. Using the scale reference markings the difference between the values observed and calculated to yield the length of the stricture This knowledge can be important in subsequent treatment, such as correct sizing of a biliary stent.

In the invention, a second system of indicia is used as an aid to the endoscopist to detect longitudinal movement of the wire guide. This can be important in that dislodgement of the wire guide, when serving as an exchange wire, can easily occur as other devices are being passed thereover. These indicia for detection of motion can include, diagonal or helical lines printed on the wire guide surface, or incorporating helical striping into the wire guide outer coating. The second system indicia can be placed on the proximal portion of the wire guide to be observed directly by the endoscopist, or they can also be placed at the distal portion of the wire guide such that they are viewable by the endoscope as the wire guide exits the accessory channel.

In still another aspect of the invention, additional scale reference markings can be placed on the distal portion of the wire guide to supplement those at the proximal end. These distally located scale reference markings, which can be numeric indicia, a sequentially increasing series of markings, or a combination thereof, are viewable by endoscope, rather than by direct observation as are those at the proximal end.

### Brief Description of the Drawing

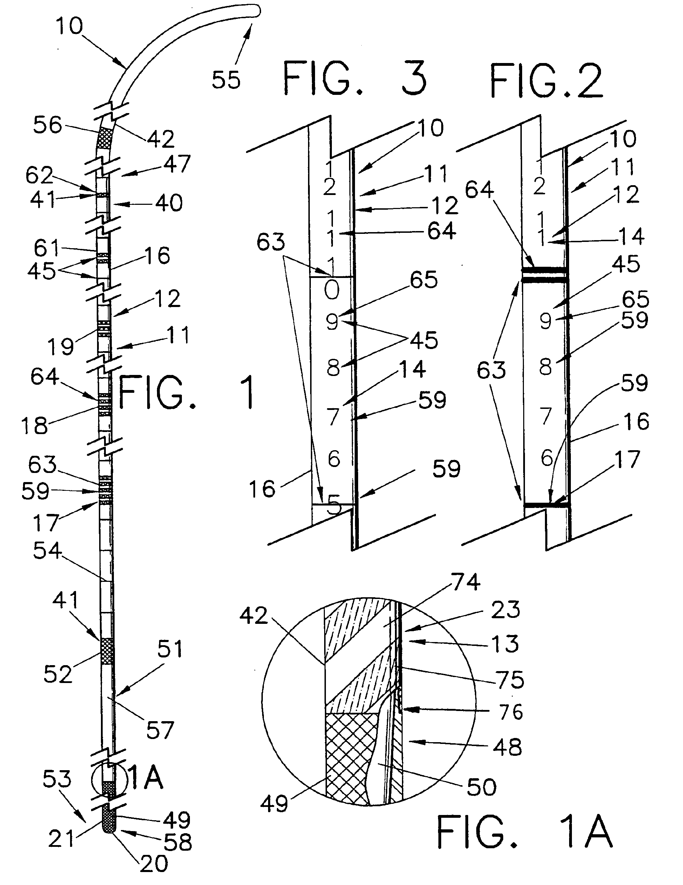

FIG. 1 depicts a side view of an embodiment of the present invention;
FIG. 1 a depicts a partially sectioned detail view of the embodiment of FIG. 1;
FIGs. 2-3 depict enlarged detail views of second and third embodiments of the present invention;
FIG. 4 depicts a side view of a fourth embodiment of the present invention;
FIG. 5 depicts an enlarged pictorial view of the embodiment of FIG. 4 being used with an endoscope;
FIG. 6 depicts a view of the device being used *in vivo;* and
FIG. 7 depicts an enlarged pictorial view of a fifth embodiment of FIG. 1 being used with an endoscope.

### Detailed Description

To better understand the following specification, definitions are provided for the following terms used herein. Referring now to terms used to reference FIGs. 1-3, fixed reference point 58 is defined as a designated point along the medical device to which the scale indicia (numbers, bands, etc.,) have a known distance relationship. This can either be the distal tip of the medical device, or a more proximal point therealong. If bands or other unique symbols are used rather than numerical values, a fixed reference point can occur at more than one location on the device. For example, if a series of non-numerical marking start at the 200 cm point from the distal end of the device, fixed reference points can be said to occur at both the distal end at the 200 cm mark, since the scale markings are referable back to both points. Scale reference markings 59 refers to the unique members of a series of indicia for indicating a distance, actual or relative, to the fixed reference point. Collectively, these are known as the first system of indicia 12, which may also include intermediate markings 54. The intermediate markings 54 comprise lines or other non-unique indicia that are provided at regular increments between adjacent scale reference markings 59. Numerical reference markings 14, an embodiment of scale reference markings 59, refers to the numerical value that corresponds to the distance from that particular point to the fixed reference point 58. Numerical scale system 65 refers to the collective numerical reference markings 14. The sequentially increasing marker series 64 refers to an embodiment of the first system of indicia 12 in which there are an increasing numbers of bands 41, dots, etc. at regular intervals (e.g., 5 cm as depicted in FIG. 1) as one moves toward or away from the fixed reference point 58. In relation to the length of the medical device 10, the distal portion 53 is defined as that part that normally exits the distal end of the endoscope during the procedure. If scale indicia are included on the wire guide for viewing by the endoscope, it is the distal portion 53 that bears these indicia. The proximal portion 40 of the wire guide is that portion that is mostly outside the proximal end of the endoscope or catheter in which the wire guide resides. This includes the first system of indicia that is directly viewable to the endoscopist. The intermediate portion 51 is situated between the distal and proximal portions 51,40 and normally does not include the first system of indicia in that it mostly inside the endoscope during the procedure. The second system of indicia 13 includes markings, usually diagonal or helical in nature, that help indicate to the operator that the device is advancing or retracting relative a stationary device, such as an endoscope. The second system of indicia may be located at the distal portion 53 for viewing by the endoscope, the proximal portion 40 for direct visual monitoring, or they may extend over the entire length of the device. The second system of indicia 13 may include both printed marking and helical striping that is incorporated into the wire guide outer coating. All other elements of the present invention will be discussed with the figures.

FIGS. 1-7 depict a medical device 10 comprising a wire guide 16 having a distal portion 53, and intermediate portion 51, and a proximal portion 40 having an indicia pattern 11 that allows the wire guide 16 to be used with an endoscope 25 to measure anatomical structures and other distances within a patient. The illustrative device 10 preferably comprises a standard exchange wire guide 16, e.g., 480 cm or 260 cm in length, with a solid core wire, such as nitinol, and an outer polymer coating 42, such as PET or another suitable material, that is heat shrink-wrapped over the wire. To aid in fluoroscopic positioning of the wire guide, a distal portion of the device contains at least one radioactive marker 21. Different methods of providing radiopacity include standard techniques such as the addition of a distal platinum coil, adding gold or other radiopaque markers, using radiopaque inks, or the use of radiopaque shrink wrap or tubing 49 over the core wire 50, e.g., radiopaque urethane, or dipping the wire in a radiopaque polymer, or gluing on a radiopaque tip 49 such one made of PEBAX^{®} that has been loaded with tungsten, barium, or some other radiopaque powder by a method such as that disclosed in U.S. Patent 5,300,048 to Drewes. The tip 49 can then be dipped in a hydrophillic material to make it more lubricious. In the illustrative embodiment, the indicia pattern 11 is printed in ink on the outer coating 42 of the wire guide, although other well-known methods of imprinting or marking medical devices could be used (e.g., chemical etching or engraving). The bands 41 are imprinted or applied to the wire guide as a separate material that can be radiopaque.

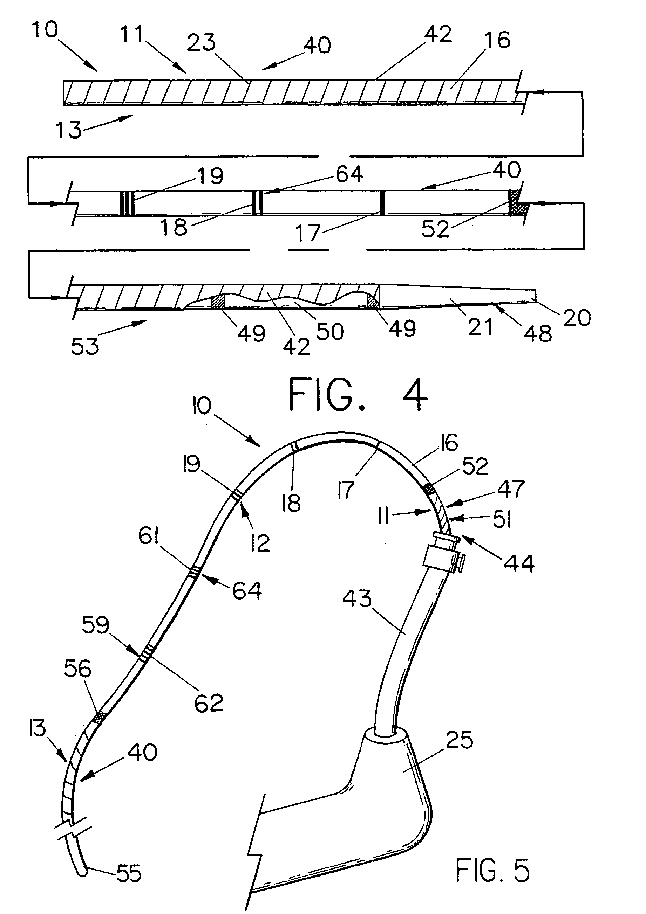

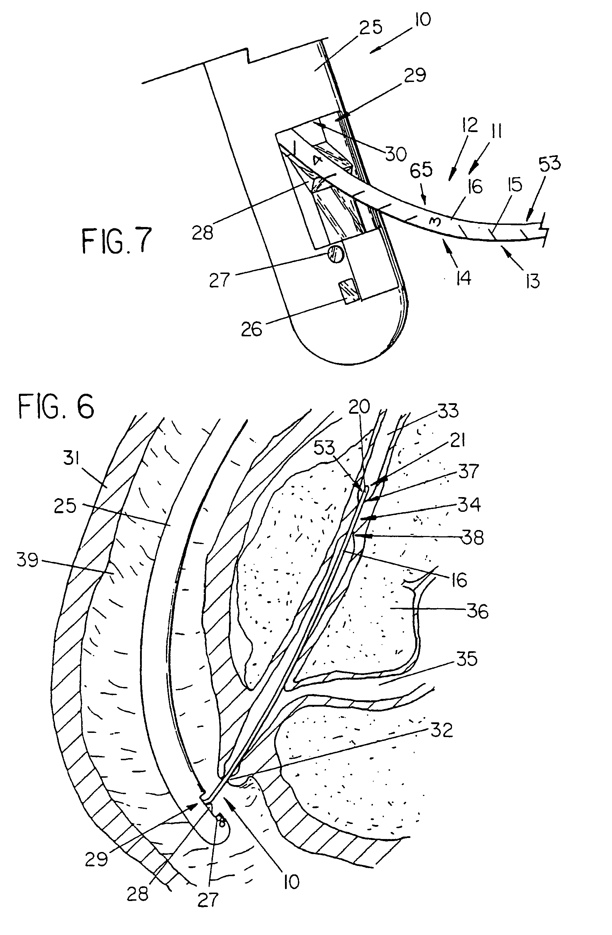

In the embodiment of FIG. 1, the indicia pattern 11 includes a first system of indicia 12 is located on the proximal portion 40 of the wire guide 16. In the illustrative embodiment, the proximal portion 40 begins at the distal boundary of the first system of indicia 12, which lie between the distal mark 52 located 200 cm from the distal tip, and the proximal mark 56 located 30 cm from the distal mark. The remainder of the proximal portion 40 does not include scale indicia in the illustrative embodiment. Within the 30 cm portion comprising the first system of indicia 12, there are a series of unique scale reference markings 59 that correspond to a particular distance to a known fixed reference point 58. The scale reference markings 59 collectively form forming the sequentially increasing marker series 64, each comprising of different number of bands 17,18,19,61,62 centered at 5 cm increments from one another at the 5, 10, 15, 20, and 25 cm points from the distal (200 cm) mark 52. Because the scale reference markings 59 in this particular embodiment are relative, rather than an absolute measurement, the fixed reference point 58, or the reference scale's "point zero", can be considered to be either the distal end 20, the distal mark 52, or the proximal mark 56. When numeric indicia 14 are used such as in FiGs. 2-3, the fixed reference point 58 is that point on the wire guide 16 that serves as the point zero of the reference scale. In this particular embodiment the first reference mark 17 comprises five bands, with the second reference mark 18 having four bands, the third reference mark 19 having three bands, the fourth reference mark 61 having two bands, and the fifth reference mark 62 having one band. Alternatively, the number of bands can decrease distally to proximally and may include fewer or a greater number of reference markings. Intermediate markings 54, comprising a series of four single lines are each located at 1 cm increments between adjacent members of the sequentially increasing marker series 64. In the illustrated embodiment, single, non-unique bands 41 serve both as the distal mark 52 and the proximal mark 56 to indicate the boundaries of the sequentially increasing marker series 64. The 30 cm portion of the wire guide having the scale reference markings 59 corresponds to that portion that would normally fall at the exit point from the endoscope during an ERCP procedure, such as when a stricture is being measured. FIG. 5 depicts the wire guide 16 of the present invention being used with an endoscope 25 with the entire sequentially increasing marker series 64 lying outside the endoscope 25 and catheter 43 within the accessory channel 30 thereof. As the wire guide 16 is advanced into the common bile duct 33, e.g., to measure a stricture 33, as depicted in FIG. 6, the point where the wire guide 16 exits the wire guide port 44 should lie well within the first system of indicia 12 such that a measurement can be obtained. FIG. 6, wire guide 16 depicts the illustrative wire guide 16 being used in an ERCP procedure to measure a stricture 34 in the common bile duct 33. The basic procedure would also be used for obtaining measurements elsewhere within the biliary system, such as the pancreatic duct 35. As depicted, the endoscope 25 is first advanced down into the duodenum to the Papilla of Vater 32, the entrance to the biliary system. The wire guide 16 is then advanced from the accessory channel 30 of the endoscope 25. The elevator 28 of the endoscope 25 is positioned to laterally deflect the wire guide 16 from the side opening 29 of the scope to facilitate advancement through the duct to the stricture 34. With the tip 20 of the wire guide 16 having a radiopaque component 21, the device is guided under fluoroscopy to the distal point (furthest from opening) 37 of the stricture 34. At that point, the operator notes the position of the proximal portion 40 of the wire guide 16 using the indicia pattern 11 printed thereon as depicted in FIG. 5. When the wire guide 16 is properly sized for the procedure, the desired situation should exist in which the point along the wire guide 16 exiting the wire guide port 44 of the endoscope 25 lies within the scale references markings 59 of the first system of indicia 12. After the first value has been determined, by noting the particular scale reference mark 59 at that location, the wire guide 16 is withdrawn until the radiopaque marker 21 (such as the wire guide tip 20) corresponds to the proximal point 38 (closest to opening) of the stricture 34. At that time, the position of the wire guide 16 is reread relative the wire guide port 44, and the difference is calculated to determine the stricture 34 length. To assist in measurement, an external marker, such as a clip, tape, etc., can be used as a reference to mark the wire guide in the initial position. Following the measurement, a catheter or other device can be advanced over the wire guide which can facilitate the exchange of different devices.

FIG. 7 depicts an embodiment of the present invention which the indicia pattern 11 includes second system of indicia 13 for helping the endoscopist better discern whether the wire guide 16 is moving relative to the endoscope 25 in which it resides. In the illustrative embodiment, the second system of indicia comprise a series of oblique markings 15 printed on the proximal portion 53 of the wire guide. These oblique markings 15 are located between numeric indicia 14 comprising first system of indicia 12. A second set of oblique markings 15 can be placed 180° with respect to each other for easier viewing through the endoscope 25. The diagonal orientation of the markings makes longitudinal movement of the wire guide 16 more obvious to the operator. Helical striping 23 as depicted in FiGs. 4-5 provides another embodiment of the second system of indicia 13 which can be located throughout the length of the wire guide for facilitation detection of movement via either the endoscope (the distal portion 53) or direct observation (the proximal portion 40). FIG. 1a depicts an further embodiment having helical striping that includes alternating colored stripes 74,75 incorporated into the polymer outer coating 42 of the wire guide 16. As a result, additional first or second system-type indicia 12,13 can be printed over the top of the helical striping 74, 75. In the illustrative embodiment, the numeric scale system 65 includes numeric indicia 14 located at 1 cm increments. An optional sequentially increasing marker series (not shown) similar to that of the proximal portion 40 of FIG. 1, can be used at 5 cm increments or as an alternative to the numeric scale system. In the embodiment of FIG. 7, the wire guide 16 with indicia pattern 11 at the distal portion 53 is positioned at the ends of the stricture, as in the embodiment of FIG. 5, at which time the numeric indicia 14 can be read via the camera lens 27 under illumination of the light source 26. The proximal and distal portion 40, 53 indicia patterns of FIGs. 1 and 7 can be advantageously combined to offer both options to the endoscopist In a single device.

Optionally, the outer coating 42 of portions 40, 51 and 53 of the wire guide can comprise different materials. For example, the distal portion 63 can have a PET coating, which is more acceptable of printing, while the intermediate portion 51 and/or proximal portion 40 can be made of PTFE, which often requires a surface pre-treatment prior to imprinting indicia thereon, In another embodiment, the distal most portion of the distal portion 53 of the device, e.g., 5 cm, is advantageously made radiopaque by loading of tungsten or barium powder to a separate polymer tip 49, such as that made of PEBAX^{®} as shown in FIG. 1 a. The tip 49, which contains a shoulder 76, is glued to the tapered section 48 of the core wire 50 using cyanoacrylate or another suitable adhesive. The outer coating 42 attaches over the shoulder 76 to produce a smooth transition. Additionally, an alternate type of first or second indicia 12,13 is depicted in FIG. 4 that includes radiopaque markers such as radiopaque tubing 49 (e.g., radiopaque urethane) applied to the core wire 50 in distal portion 53 with the coating 42 placed thereover. As depicted, the radiopaque tubing 49 provides a series of radiopaque indicia at regular intervals that correspond to a known scale for determining length (e.g., 5 cm intervals).

It should be understood that although the illustrative embodiments include a wire guide having indicia, the indicia patterns described herein can be applied to any elongated medical device that might be used with an endoscope, such as a catheter, sphincterotome, or other related device.

## Claims

1. A medical device comprising an elongate member (16) that includes a distal tip, a distal portion (53), and a proximal portion (40), and an indicia pattern (11), wherein the indicia pattern (11) includes a first system of indicia; wherein at least a portion of the first system of indicia located on the proximal portion (40) is viewable to an operator of the medical device, the elongate member (16) comprising a wire guide that includes the first system of indicia, wherein the first system of indicia includes scale reference markings (59) comprising a series of unique markings referenced to a fixed reference point on the device; **characterised in that** the indicia pattern includes a second system (13) of indicia that includes a pattern of markings located at least partially between selected ones of the scale reference markings (59), whereby the second system (13) of indicia comprises a series of discrete diagonal lines (15) that at least partially circumscribe the elongated member (16), and/or at least one helical stripe (23) extending along at least a portion of the elongated member (16), and/or an outer layer, whereby the outer layer including alternating first and second helical striping incorporated thereinto.

2. The device of claim 1, wherein the scale reference markings comprise a sequentially increasing marker series (17-62, 64) at distributed along the proximal portion (40) of the wire guide at a selected interval.

3. The device of claim 2, wherein the selected interval is 5 cm.

4. The device of claim 1, wherein the scale reference markings (64) include numerical values (64) identifying the particular distance to the fixed reference point.

5. The device of claim 1, wherein the first system of indicia further includes a plurality of intermediate markings (54) located therebetween selected adjacent scale reference markings.

6. The device of claim 5, wherein the scale reference markings (59) are located at 5 cm increments relative to a fixed reference point located along the wire guide and the intermediate markings (54) are located at 1 cm increments relative to the fixed reference point.

7. The device of claim 1, wherein the distal portion includes at least one radiopaque marker (58).

8. The device of claim 7, wherein one of the at least one radiopaque marker (58) includes the distal tip.

## Patentansprüche

1. Medizinische Vorrichtung mit einem länglichen Element (16), das eine distale Spitze, einen distalen Abschnitt (53) und einen proximalen Abschnitt (40) aufweist, und einem Markierungsmuster (11), worin das Markierungsmuster (11) ein erstes System von Markierungen aufweist; worin zumindest ein Teil des ersten Systems von Markierungen, der auf dem proximalen Abschnitt (40) angeordnet ist, für einen Bediener der medizinischen Vorrichtung sichtbar ist, wobei das längliche Element (16) einen Führungsdraht aufweist, der das erste System von Markierungen aufweist, worin das erste System von Markierungen Skalenreferenzmarkierungen (59) aufweist, die eine Reihe von einzigartigen Markierungen umfassen, die sich auf einen fixen Bezugspunkt auf der Vorrichtung beziehen; **dadurch gekennzeichnet, dass** das Markierungsmuster ein zweites System (13) von Markierungen aufweist, das ein Muster von Markierungen aufweist, die zumindest teilweise zwischen ausgewählten Skalenreferenzmarkierungen (59) angeordnet sind, wobei das zweite System (13) von Markierungen eine Reihe von diskreten diagonalen Linien (15) umfasst, die zumindest teilweise das längliche Element (16) umschreiben, und/oder zumindest einen spiralförmigen Streifen (23), der sich zumindest über einen Teil des länglichen Elements (16) erstreckt, und/oder eine Außenschicht umfasst, wobei die Außenschicht abwechselnde, in sie eingearbeitete erste und zweite spiralförmige Streifen aufweist.

2. Vorrichtung nach Anspruch 1, worin die Skalenreferenzmarkierungen eine sequentiell steigende Reihe von Markierungen (17-62, 64) aufweist, die über den proximalen Abschnitt (40) des Führungsdrahts in ausgewählten Abständen verteilt sind.

3. Vorrichtung nach Anspruch 2, worin der ausgewählte Abstand 5 cm beträgt.

4. Vorrichtung nach Anspruch 1m, worin die Skalenreferenzmarkierungen (64) numerische Werte (64) aufweisen, die den jeweiligen Abstand zu dem fixen Bezugspunkt kennzeichnen.

5. Vorrichtung nach Anspruch 1, worin das erste System von Markierungen ferner eine Vielzahl von Zwischenmarkierungen (54) aufweist, die zwischen ausgewählten benachbarten Skalenreferenzmarkierungen angeordnet sind.

6. Vorrichtung nach Anspruch 5, worin die Skalenreferenzmarkierungen (59) in 5-cm-Schritten relativ zu einem fixen Bezugspunkt auf dem Führungsdraht angeordnet sind und worin die Zwischenmarkierungen (54) in 1-cm-Schritten relativ zu dem fixen Bezugspunkt angeordnet sind.

7. Vorrichtung nach Anspruch 1, worin der distale Abschnitt zumindest eine röntgendichte Markierung (58) aufweist.

8. Vorrichtung nach Anspruch 7, worin eine der zumindest einen röntgendichten Markierungen (58) die distale Spitze aufweist.

## Revendications

1. Dispositif médical constitué d'un élément allongé (16) qui inclut une pointe distale, une partie distale (53), et une partie proximale (40), et un motif d'inscriptions (11), dans lequel le motif d'inscriptions (11) inclut un premier système d'inscriptions ; dans lequel au moins une partie du premier système d'inscriptions positionnée sur la partie proximale (40) est visible par un opérateur du dispositif médical, l'élément allongé (16) comportant un guide-fil qui inclut le premier système d'inscriptions, dans lequel le premier système d'inscriptions inclut des repères de référence gradués (59) présentant une série de repères uniques référencés à un point de référence fixe sur le dispositif ; **caractérisé en ce que** le motif d'inscriptions inclut un second système (13) d'inscriptions qui inclut un motif de repères positionné au moins partiellement entre une partie choisie des repères de référence gradués (59), ledit second système (13) d'inscriptions comportant une série de lignes diagonales discrètes (15) qui circonscrivent au moins partiellement l'élément allongé (16), et/ou au moins une raie hélicoïdale (23) s'étendant le long d'au moins une partie de l'élément allongé (16), et/ou une couche externe, ladite couche externe incorporant alternance d'une première et seconde raie hélicoïdale y étant incorporée.

2. Dispositif selon la revendication 1, dans lequel les repères de référence gradués comportent une série de marqueurs à progression séquentielle (17-62, 64) distribués le long de la partie proximale (40) du guide-fil à un intervalle choisi.

3. Dispositif selon la revendication 2, dans lequel l'intervalle choisi est de 5 cm.

4. Dispositif selon la revendication 1, dans lequel les repères de référence gradués (64) incluent des valeurs numériques (64) identifiant la distance particulière au point de référence fixe.

5. Dispositif selon la revendication 1, dans lequel le premier système d'inscriptions inclut en outre une pluralité de repères intermédiaires (54) positionnés entre les repères de référence gradués adjacents choisis.

6. Dispositif selon la revendication 5, dans lequel les repères de référence gradués (59) sont positionnés par incréments de 5 cm par rapport à un point de référence fixe positionné le long du guide-fil et les repères intermédiaires (54) sont positionnés par incréments de 1 cm par rapport au point de référence fixe.

7. Dispositif selon la revendication 1, dans lequel la partie distale inclut au moins un marqueur radio-opaque (58).

8. Dispositif selon la revendication 7, dans lequel l'un dudit ou desdits marqueurs radio-opaques (58) inclut la pointe distale.
